# EUROPÄISCHE PATENTSCHRIFT

(11) **EP 2 150 540 B1**
(45) Veröffentlichungstag und Bekanntmachung des Hinweises auf die Patenterteilung: **01.06.2016**
(21) Anmeldenummer: 08733252.4
(22) Anmeldetag: 24.04.2008
(51) Int. Cl.: C07D 295/108, A61K 31/4453

(54) **VERFAHREN ZUR HERSTELLUNG VON HOCH REINEM 2,4 '-DIMETHYL-3-PIPERIDINO-PROPIOPHENON (TOLPERISON), DIESES ENTHALTENDE PHARMAZEUTISCHE ZUSAMMENSETZUNGEN, SOWIE TOLPERISON ENTHALTENDE WIRKSTOFFFORMULIERUNGEN**
METHOD FOR THE PRODUCTION OF HIGHLY PURE 2,4'-DIMETHYL-3-PIPERIDINO-PROPIOPHENONE (TOLPERISONE), PHARMACEUTICAL COMPOSITIONS CONTAINING THE SAME, AND AGENT FORMULATIONS CONTAINING TOLPERISONE
PROCÉDÉ DE PRODUCTION DE 2,4'-DIMÉTHYL-3-PIPÉRIDINO-PROPIOPHÉNONE (TOLPÉRISONE) DE HAUTE PURETÉ, COMPOSITIONS PHARMACEUTIQUES RENFERMANT CE COMPOSÉ, ET FORMULATIONS DE PRINCIPE ACTIF RENFERMANT DE LA TOLPÉRISONE

(30) Priorität: 26.04.2007 AT 6582007; 29.11.2007 AT 19532007; 08.02.2008 US 27287
(43) Veröffentlichungstag der Anmeldung: 10.02.2010
(73) Patentinhaber: Sanochemia Pharmazeutika AG, 1090 Wien (AT)
(72) Erfinder: WELZIG, Stefan, A-1030 Wien (AT); ROTHENBURGER, Jan, A-7064 Oslip (AT); KÄLZ, Beate, A-7035 Steinbrunn (AT); GUNGL, József, H-9400 Sopron (HU); GERDES, Klaus, D-40233 Düsseldorf (AT); GAETA, Federico, Mountain View, CA 94040-1334 (US)
(74) Vertreter: Beer & Partner Patentanwälte KG
(86) Internationale Anmeldenummer: PCT/AT2008/000149
(87) Internationale Veröffentlichungsnummer: WO 2008/131469

(56) Entgegenhaltungen:
- WO-A-2004/050648
- WO-A-2005/084676

## Beschreibung

Die Erfindung betrifft ein Verfahren zur Herstellung von hoch reinem 2,4'-Dimethyl-3-piperidino-propiophenon (Tolperison), seiner pharmazeutisch annehmbaren Salze, Hydrochloride und Hydrate.

Tolperison ist ein muskelentspannendes Mittel (Muskelrelaxans) mit der nachstehenden Formel

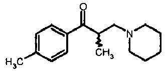

Hauptindikationen von Tolperison sind Erkrankungen, die mit schmerzhaften Muskelverspannungen einhergehen, z.B. Wirbelsäulensyndrome, muskuläre Schmerzen bei degenerativen Erkrankungen, berufs- und sportbedingte Überlastungssyndrome und das Fibromyalgiesyndrom.

Ein Vorteil der Behandlung mit Tolperison ist, dass auch funktionelle Parameter, wie z.B. die Mobilität des Patienten, verbessert werden. Patienten mit langfristiger Einnahme von Tolperison haben durch das Fehlen zentraler Nebenwirkungen in der Regel ein gutes therapeutisches Verhältnis und die zum Therapieerfolg notwendige Vertrauensbasis zum weiteren Einsatz dieses Medikaments. Eine Voraussetzung dafür ist, dass der Wirkstoff Tolperison in möglichst reiner Form hergestellt, und somit der Anteil von unerwünschten Nebenprodukten so gering wie möglich gehalten wird.

Verfahren zur Herstellung von Tolperison sind bekannt aus: AT 413 539 bzw. WO-A-2004/050648, U.S. Patent Application Publication No. 20060041141; Ditriech et al. (1999) J. Labeled Cpd. Radiopharm 42:1125-1134; Jap. Pat. 04005283 19920109; Jap. Pat. 54032480 19790309; Jap. Pat. 54036274 19790316; Jap. Pat. 54030178 19790306; Jap. Pat. 54027571 19790301; Kazuharu et al. (1994) Chem. Pharm. Bulletin 42(8) 1676; Jap. Pat. 20,390 (1965); and Hung. Pat. 144,997 (1956).

Diese beschreiben jedoch alle keinen Weg, der ein Verfahren zur dauerhaften Abreicherung und Stabilisierung, der besonders kritischen Verunreinigung 2-methyl-1-(4-methylphenyl)-propenon (4-MMPPO) ermöglicht, noch wird ein Verfahren zur Abreicherung der Verunreinigung 2-methyl-1-(4-methylphenyl)-propenon (4-MMPPO) in den ppm-Bereich beschrieben, welcher von der FDA und EMEA vorgeschrieben wird.

Grundsätzlicher Nachteil dieser Synthesewege ist es, dass das gewonnene, Tolperison enthaltende Endprodukt, aufgrund von Nebenreaktionen und chemischen Verunreinigungen unerwünschte Stoffe, insbesondere 4-MMPPO, in solchen Konzentrationen enthält und unter Lagerbedingungen bilden kann, die toxisch für mit Tolperison behandelte Patienten sind.

Abhängig von der verwendeten Synthesemethode werden folgende Verunreinigungen im Wirkstoff Tolperison gefunden: Piperidinhydrochlorid, 2-Methyl-1-(3- methylphenyl)-3-(1-piperidinyl)-propanon hydrochlorid (3-Tolperisonhydrochlorid), 1-(4-Methylphenyl)-propanon (4-Methylpropiophenon), 2-Methyl-1-(4-methylphenyl)-propenon (4-MMPPO), sowie 2-Methyl-1-(2-methylphenyl)-3-(1-piperidinyl)-propanon hydrochlorid (2-Tolperisonhydrochlorid).

Eine Übersicht betreffend möglicher Verunreinigungen ist folgender Tabelle zu entnehmen:

| Bezeichnung | Chemischer Name | Chemische Struktur |
|---|---|---|
| Piperidin HCl | Piperidinhydrochlorid | 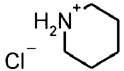 |
| C | 2-Methyl-1-(3-methylphenyl)-3-(1-piperidinyl)-propanon-hydrochlorid oder 3-Tolperisonhydrochlorid | 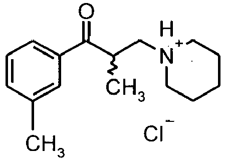 |
| 4-MPP | 1-(4-Methylphenyl)-propanon oder 4-Methylpropiophenon | 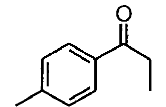 |
| 4-MMPPO | 2-Methyl-1-(4-methylphenyl)-propenon | 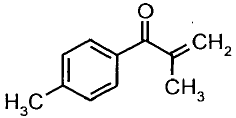 |
| D | 2-Methyl-1-(2-methylphenyl)-3-(1-piperidinyl)-propanpon hydrochlorid oder 2-Tolperisonhydrochlorid | 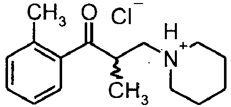 |

Auf die Verunreinigung 2-Methyl-1-(4-methylphenyl)-propenon (4-MMPPO) muss dabei ein besonderes Augenmerk gelegt werden, da diese auf Grund ihrer chemischen Struktur und präklinischer in vitro Tests als potentiell genotoxisch eingestuft werden muss. Neueste Guidelines der FDA und EMEA bezüglich genotoxischer Substanzen (Guideline on the limits of genotoxic impurities CPMP/SWP/5199/02) definieren den maximalen Gehalt dieser Substanzen von < 1,5 ig/Tag in arzneilichen Wirkstoffen, was bei behandlungsüblichen Dosierungen von Tolperison einen potentiellen Grenzwert zwischen maximal 25 und 1 ppm an 4-MMPPO im Wirkstoff und Fertigprodukt zur Folge hat. Eine Zulassung der FDA für eine Tolperison-Formulierung ist nur dann möglich, wenn diese Grenzwerte unterschritten werden.

Unabhängig von der jeweiligen, bisher bekannten Synthese entsteht immer das bekannte, unerwünschte Nebenprodukt 2-Methyl-1-(4-methylphenyl)-propenon (4-MMPPO) mit folgender Formel:

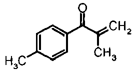

Das Nebenprodukt 4-MMPPO wird aus Tolperison durch beta-Eliminierung gebildet:

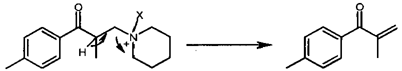

Diese unerwünschte Nebenreaktion erfolgt sowohl bei der Herstellung von Tolperison als auch bei dessen Lagerung. Dies gilt sowohl für den Wirkstoff als auch für arzneiliche Formulierungen, bei deren Herstellung und Lagerung ebenfalls 4-MMPPO als Abbauprodukt entstehen kann. Eine Übersicht gibt folgende Tabelle über 4-MMPPO Konzentrationen in käuflichen Tolperison-Formulierungen:

| Probe | Produkt | Lot | Herstelldatum | 4-MMPPO in ppm |
|---|---|---|---|---|
| 1 | Mydeton | T666T2A | Jul-06 | 215 |
| 2 | Mydeton | T6A207A | Oct-06 | 107 |
| 3 | Mydocalm | 506223 | May-05 | 442 |

Eine Aufgabe der vorliegenden Erfindung ist es daher, ein Verfahren zur Herstellung von hoch reinem 2,4'-Dimethyl-3-piperidino-propiophenon (Tolperison), seiner pharmazeutisch annehmbaren Salze, Hydrochloride und Hydrate vorzuschlagen, mit welchem der Anteil an 2-Methyl-1-(4-methylphenyl)-propenon (4-MMPPO) jedenfalls unter den von FDA und EMEA geforderten Höchstwerten erzielt wird.

Erfindungsgemäß wird ein Verfahren der eingangs genannten Art vorgeschlagen, bei dem 4-Methylpropiophenon 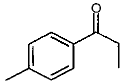 mit Piperidinhydrochlorid 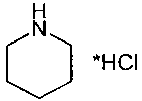 und 1,3-Dioxolan 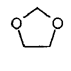 in Gegenwart einer oder mehrerer Säuren als Katalysator umgesetzt wird und Tolperison als Rohprodukt in freier Form oder in Form seiner pharmazeutisch verträglichen Hydrochloride, Hydrate und Additionssalze nach dem Abkühlen aus der Reaktionsmischung abfiltriert wird und wobei durch Umkristallisieren des Tolperison-Rohproduktes der Anteil an 2-Methyl-1-(4-methylphenyl)-propenon (4-MMPPO) reduziert wird. Das erfindungsgemäße Verfahren ist dadurch gekennzeichnet, dass beim Umkristallisieren des Tolperison-Rohproduktes eine Säure zugesetzt und der Anteil an 2-Methyl-1-(4-methylphenyl)-propenon (4-MMPPO) auf weniger als 10 ppm bezogen auf 100 Gew.% % Tolperison-Rohprodukt reduziert wird. Mit dem erfindungsgemäßen Verfahren kann der Anteil an 4-MMPPO erheblich reduziert und insbesondere auf "Null" verkleinert werden, so dass das erfindungsgemäß erhältliche Tolperison kein 4-MMPPO bzw. 4-MMPPO in Anteilen nur unterhalb der Nachweisgrenze enthält.

Beispielsweise kann der Anteil des erfindungsgemäß erhältlichen Tolperison an 4-MMPPO auf weniger als 0,15 Gew.% bezogen auf 100 Gew.% Rohprodukt verringert werden.

Das erfindungsgemäße Verfahren erlaubt es auch, dass der Anteil an 2-Methyl-1-(4-methylphenyl)-propenon (4-MMPPO) auf weniger als 0,15 Gew.%, insbesondere auf weniger als 0,002 Gew.% bezogen auf 100 Gew.% Tolperison-Rohprodukt reduziert wird. Dabei ist es möglich, dass der Anteil an 2-Methyl-1-(4-methylphenyl)-propenon (4-MMPPO) auf weniger als 10 ppm reduziert wird. Insbesondere kann der Anteil an 2-Methyl-1-(4-methylphenyl)-propenon (4-MMPPO) auf weniger als 7 ppm oder auf weniger als 3 ppm reduziert werden kann. Schließlich erlaubt es das erfindungsgemäße Verfahren, dass der Anteil an 2-Methyl-1-(4-methylphenyl)-propenon (4-MMPPO) auf weniger als 1,5 ppm oder auf 0 ppm (das heißt unterhalb der Nachweisgrenze) reduziert wird.

Weitere vorteilhafte Ausgestaltungen dieses Verfahrens sind gemäß Unteransprüche offenbart.

Des Weiteren ermöglicht das erfindungsgemäße Verfahren den Anteil an der Verunreinigung Piperidinhydrochlorid, der sowohl als Rohstoff eingesetzt wird, als auch ein zusammen mit 4-MMPPO entstehendes Abbauprodukt ist, auf Werte unterhalb von 100 ppm bis 0 ppm (Nachweisgrenze) abzusenken.

Das erfindungsgemäß hergestellte Tolperison kann für pharmazeutische Formulierungen, welche den Wirkstoff Tolperison in freier Form bzw. in Form seiner pharmazeutisch annehmbaren Salze, Hydrochloride und Hydrate in Verbindung mit einem oder mehreren pharmazeutisch verträglichen Träger(n) enthalten, verwendet werden. Mögliche Verabreichungsformen sind Tabletten, Kapseln, Granalien, Suspensionen, Sirupe, Gele, Salben und Cremes sowie transdermale therapeutische Systeme, wie Wirkstoffpflaster.

Dabei kann als Wirkstoff Tolperison in reiner Form, in Form seiner Salze, Hydrochloride oder Hydrate sowie 2-Methyl-1-(4-methylphenyl)-propenon (4-MMPPO) in einem Anteil von weniger als 10 ppm, von weniger als 7 ppm, von weniger als 3 ppm oder 0 ppm (das heißt unterhalb der Nachweisgrenze) bezogen auf 100 Gew.% Wirkstoff eingesetzt werden.

Als Tabletten werden beispielsweise Filmtabletten mit Instant-Release Eigenschaften vorgeschlagen, bei welchen der Wirkstoff Tolperison, dessen Salze, Hydrochloride und Hydrate in Verbindung mit einem pharmazeutischen Träger und zumindest einem Additiv, nämlich eine pharmazeutisch verträgliche Säure, vorzugsweise wasserfreie Zitronensäure, vorliegt. Zur Herstellung dieser Filmtablette wird unter wasserfreien Bedingungen mit Hilfe von Lösungsmitteln gearbeitet.

Eine weitere pharmazeutische Formulierung kann dann bereitgestellt werden, wenn der Wirkstoff Tolperison in einer Matrix eingebettet vorliegt, sodass eine gezielte Wirkstofffreisetzung (controlled release) erzielt wird.

Eine weitere pharmazeutische Formulierung kann dann bereit gestellt werden, wenn die Formulierung enthaltend den nach dem erfindungsgemäßen Verfahren hergestellten Wirkstoff Tolperison, dessen Salze, Hydrochloride und Hydrate auf Basis einer Mischung aus Carbomer, Natriumhydroxidlösung, einem Alkohol und Wasser unter Zusatz einer stabilisierenden, pharmazeutisch verträglichen Säure, vorzugsweise Zitronensäure, unter Bildung eines Gels hergestellt wird.

Eine weitere pharmazeutische Formulierung kann dann bereit gestellt werden, wenn die Formulierung enthaltend den nach dem erfindungsgemäßen Verfahren hergestellten Wirkstoff Tolperison, dessen Salze, Hydrochloride und Hydrate auf Basis einer Mischung aus Cetylstearylalkohol, Wollwachsalkoholen und Vaseline unter Zusatz einer stabilisierenden pharmazeutisch verträglichen Säure, vorzugsweise Zitronensäure, unter Bildung einer Salbe hergestellt wird.

Eine weitere pharmazeutische Formulierung kann dann bereit gestellt werden, wenn die Formulierung enthaltend den nach dem erfindungsgemäßen Verfahren hergestellten Wirkstoff Tolperison, dessen Salze, Hydrochloride und Hydrate auf Basis einer Mischung aus Polysorbat, Cetylstearylalkohol, Glycerol, Vaseline, Wollwachsalkoholen und Wasser unter Zusatz einer stabilisierenden pharmazeutisch verträglichen Säure, vorzugsweise Zitronensäure, unter Bildung einer Creme hergestellt wird.

Da Tolperison als Muskelrelaxans bei chronischen Erkrankungen wie Multiple Sklerose (MS) eingesetzt wird, besteht ein Bedarf an Verabreichungsformen, die eine langsame, dauerhafte Freisetzung des Wirkstoffes gewährleisten. Ein TTS, ein transdermales therapeutisches System ist hier die geeignete Verabreichungsform.

Das TTS besteht beispielsweise aus einer Wirkstoff undurchlässigen Rückseite, beispielsweise eine Polymerfolie oder Metallfolie, vorzugsweise aus Aluminium. Die auf der Rückseite angebrachte Reservoirschicht besteht aus einer Polymermatrix und dem darin eingebetteten Wirkstoff. Der erfindungsgemäß hergestellte Wirkstoff Tolperison kann in reiner Form, in Form seiner Salze, Hydrochloride oder Hydrate sowie 2-Methyl-1-(4-methylphenyl)-propenon (4-MMPPO) in einem Anteil von weniger als 10 ppm, von weniger als 7 ppm, von weniger als 3 ppm oder 0 ppm (das heißt unterhalb der Nachweisgrenze) bezogen auf 100 Gew.% Wirkstoff eingesetzt werden.

Die Polymermatrix besteht beispielsweise aus einem Grundpolymer und üblichen Zusatzstoffen. Beispielhafte Polymere sind Kautschuk, synthetische Homo-, Block- und Co-Polymere, Polyacrylate, Polyurethane und Silicone.

Dem Grundpolymer können Weichmacher, wie höhere Alkohole, Glyceride sowie polyethoxylierte Alkohole zugesetzt werden.

Als Penetrationshilfe können physiologisch unbedenkliche Carbonsäuren eingesetzt werden.

Ein transdermales, therapeutisches System wird beispielsweise dadurch hergestellt, dass der Wirkstoff zusammen mit den Bestandteilen der haftklebenden Reservoirschicht, gegebenenfalls in Lösung homogen vermischt, auf die Wirkstoff undurchlässige Rückseite aufgestrichen wird, worauf gegebenenfalls das oder die Lösungsmittel entfernt werden. Anschließend wird die Klebeschicht mit einer entsprechenden Schutzschicht versehen.

Ein Verfahren zur Herstellung von pharmazeutischen Formulierungen enthaltend den nach dem erfindungsgemäßen Verfahren hergestellten Wirkstoff Tolperison, dessen Salze, Hydrochloride und Hydrate, wird beispielsweise derart durchgeführt, dass man den Wirkstoff unter wasserfreien Bedingungen in einem oder mehreren Lösungsmittel(n) mit zumindest einem wasserfreien Additiv, wie eine pharmazeutisch verträgliche Säure, vorzugsweise Zitronensäure vermengt, auf den pharmazeutischen Träger aufbringt und in die jeweils gewünschte Verabreichungsform überführt. Die Verwendung von Säure sowie das Arbeiten unter wasserfreien Bedingungen gewährleisten eine Abreicherung des 4-MMPPO Gehaltes während der Formulierungsherstellung sowie auch eine Stabilisierung des Produktes während der Lagerung.

Bei der Herstellung einer Tolperison enthaltenden Wirkstoffformulierung kann ein saurer Stabilisator zugesetzt werden, da bei der Lagerung der Wirkstoffformulierung die Gefahr besteht, dass in Gegenwart einer Base 2-Methyl-1-(4-methylphenyl)-propenon (4-MMPPO) gemäß folgendem Reaktionsschema gebildet wird:

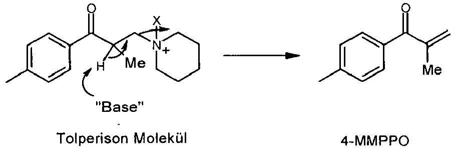

Wird der Wirkstoffformulierung ein saurer Stabilisator, wie eine für den menschlichen Körper verträgliche anorganische oder organische Säure als Stabilisator zugesetzt, so kann diese unerwünschte Nebenreaktion verhindert werden:

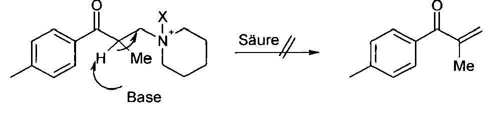

Die Erfindung wird im Folgenden anhand möglicher Ausführungsformen zur Durchführung der Erfindung erläutert.

Beispiele für mögliche Ausführungsformen der Erfindung und Vergleichsbeispiele zum Stand der Technik gemäß der AT 413 539 sind folgender Tabelle zu entnehmen:

| Variante | Umkristallisationsbedingungen | Waschbedingungen | Trocknen | 4-MMPPO in ppm |
|---|---|---|---|---|
| Stand der Technik | Erhitzen in MEK/IPA | Waschen mit MEK | 45-85°C | = 500 ppm |
| I = Vergleichs beispiel | Erhitzen MEK/IPA + Adsorptionsmittel + 2. Umkristallisieren | Waschen mit MEK | 45-85°C | = 130 ppm |
| II | Erhitzen in MEK/IPA | Waschen mit IPA mit 1% HCl | 45-85°C | ≤ 10 ppm, d.h. 1,5 bis 10 ppm |
| III | Erhitzen in MEK/IPA + 1% HCl | Waschen mit MEK | 45-85°C | ≤ 10 ppm, d.h. 0 (nicht nachweisbar) bis 10 ppm |
| IIIa | Erhitzen in MEK/IPA + 1% HCl + 5% Zitronensäure | Waschen mit MEK | 45-85°C | ≤ 10 ppm, d.h. 0 (nicht nachweisbar) bis 10 ppm |
| IV | Erhitzen in MEK/IPA + 1% HCl+ 5% Zitronensäure | Waschen mit MEK +1% HCl + 5% Zitronensäure | 45-85°C | 0 (nicht nachweisbar) bis 8 ppm |
| V | Erhitzen in MEK/IPA + 1% HCl + 5% Zitronensäure | 1. Waschen mit MEK +1% HCl + 5% Zitronensäure 2.Waschen mit MtBE | 50°C | 0 (nicht nachweisbar) bis 5 ppm |
| Va | Erhitzen in MEK/IPA + 1% HCl + 5% Zitronensäure | 1. Waschen mit MEK +1% HCl 2.Waschen mit MtBE + 5% Zitronensäure | 50°C | 0 (nicht nachweisbar) bis 1 ppm |

Das Tolperison-Rohprodukt wird nach folgendem Syntheseschema hergestellt:

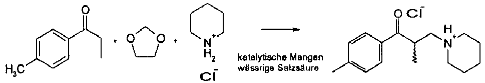

Als Ausgangsmaterial werden 4-Methylpropiophenon, Piperidinhydrochlorid und 1,3-Dioxolan als Reaktionspartner und auch als Lösungsmittel eingesetzt. Das Verwenden von 1,3-Dioxolan an Stelle von Formaldehyd und die hohe Ausbeute nach der direkten Isolierung des Tolperison-Rohproduktes machen die einstufige Reaktion auch im Industriemaßstab wirtschaftlich.

Die Reinigung des Tolperison-Rohproduktes durch Umkristallisieren wird anhand folgender möglicher Varianten und anhand von Vergleichsbeispielen gemäß Stand der Technik näher erläutert:
Stand der Technik gemäß AT PS 413 539: Das Tolperison-Rohprodukt wird in einer 85:15 Mischung aus 2-Butanon (MEK) und Isopropanol unter Rückfluss für 30 Minuten gelöst. Die Temperatur wird auf 80°C gesenkt, und die Lösung heiß filtriert. Die Lösung wird auf 5°C abkühlen gelassen und 7h gerührt. Der erhaltene kristalline Niederschlag wird abfiltriert, mit 2-Butanon gewaschen und anschließend im Vakuum bei 45-85°C getrocknet.

Durch Analyse mittels HPLC-MS/MS wird ein Anteil von 500 ppm 4-MMPPO nachgewiesen. Das Verfahren liefert somit ein Tolperison-Endprodukt, das bei längerer Lagerung noch zusätzlich steigende 4-MMPPO Werte aufweist.

Variante I) beschreibt ein Vergleichsbeispiel, worin eine ein- bzw. mehrmalige Umkristallisation, allerdings ohne Säurezusatz, durchgeführt wird.

Das Tolperison-Rohprodukt wird in einer 85:15 Mischung aus 2-Butanon (MEK) und Isopropanol unter Rückfluss für 30 Minuten gelöst. Die Temperatur wird auf 80°C gesenkt, und die Lösung heiß filtriert. Die Lösung wird auf 5°C abkühlen gelassen und 7h gerührt. Der erhaltene kristalline Niederschlag wird abfiltriert, mit 2-Butanon gewaschen und anschließend im Vakuum bei 45-85°C getrocknet. Das Abfiltrieren kann unter Beigabe eines Adsorptionsmittels, wie Aktivkohle oder Silicagel, erfolgen.

Durch Analyse mittels HPLC-MS/MS wird ein Anteil von 0,14 Gew.% 4-MMPPO nachgewiesen, welcher durch eine weitere Umkristallisation auf 130 ppm reduziert werden konnte.

Erfindungsgemäße Variante II): Umkristallisation und Waschen mit 1%-iger HCl-Isopropanol-Mischung:
Das Tolperison-Rohprodukt wird in einer 85:15 Mischung aus 2-Butanon (MEK) und Isopropanol unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert und mit 1%-iger HCl-Isopropanol-Mischung gewaschen und anschließend im Vakuum bei 45 bis 85°C getrocknet.

Durch Analyse mittels HPLC-MS/MS wird ein Anteil von 4-MMPPO in einem Bereich von 1,5 bis 10 ppm nachgewiesen.

Erfindungsgemäße Variante III) Umkristallisation mit 1%-iger HCl-IPA-MEK Mischung:
Das Tolperison-Rohprodukt wird in einer 85:15 Mischung aus 2-Butanon (MEK) und Isopropanol und unter Zugabe von 1%-iger HCl unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert, mit MEK gewaschen und anschließend im Vakuum bei 45 bis 85°C getrocknet.

Durch Analyse mittels HPLC-MS/MS wird ein Anteil von 4-MMPPO in einem Bereich von 0 ppm (nicht nachweisbar) bis 10 ppm ermittelt.

Erfindungsgemäße Variante IIIa) Umkristallisation mit 1%-iger HCl-IPA-MEK + 5% Zitronensäure Mischung:
Das Tolperison-Rohprodukt wird in einer 85:15 Mischung aus 2-Butanon (MEK) und Isopropanol und unter Zugabe von 1%-iger HCl und 5% Zitronensäure unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert, mit MEK gewaschen und anschließend im Vakuum bei 45° bis 85°C getrocknet.

Durch Analyse mittels HPLC-MS/MS wird ein Anteil von 4-MMPPO in einem Bereich von 0 ppm (nicht nachweisbar) bis 10 ppm ermittelt. Diese Verfahrensvariante zeigt den besonderen Vorteil, dass Zitronensäure im Tolperison-Endprodukt vorhanden ist, und demgemäß ein stabilisierender Effekt nachgewiesen werden konnte.

Erfindungsgemäße Variante IV) Umkristallisation mit 1%-iger HCl-IPA-MEK Mischung plus 5% Zitronensäure und anschließendem Waschen mit Säurezusatz:
Das Tolperison-Rohprodukt wird in einer 85:15 Mischung aus 2-Butanon (MEK) und Isopropanol (IPA) sowie unter Zugabe von 1%-iger HCl und 5 Gew.% (bezogen auf den Wirkstoff) Zitronensäure unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert, mit MEK, 0,5% HCl und 5% Zitronensäure gewaschen und anschließend im Vakuum bei 45 bis 85°C getrocknet.

Durch Analyse mittels HPLC-MS/MS wird ein Anteil von 4-MMPPO in einem Bereich von 0 ppm (nicht nachweisbar) bis 8 ppm ermittelt.

Erfindungsgemäße Variante V) Umkristallisation mit 1%-iger HCl-IPA-MEK Mischung plus 5% Zitronensäure und zusätzlichem Waschen mit leichtflüchtigem etherischen Lösungsmittel:
Das Tolperison-Rohprodukt wird in einer 85:15 Mischung aus 2-Butanon (MEK) und Isopropanol (IPA) und unter Zugabe von 1%-iger HCl und 5 Gew.% Zitronensäure unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert, mit MEK, 0,5% HCl und 5% Zitronensäure gewaschen, ein weiteres Mal mit der gleichen Volumenmenge an tert-Butylmethylether gewaschen und anschließend im Vakuum bei 45 bis 85°C getrocknet.

Durch Analyse mittels HPLC-MS/MS wird ein Anteil von 4-MMPPO in einem Bereich von 0 ppm (nicht nachweisbar) bis 10 ppm ermittelt.

Erfindungsgemäße Variante Va) Umkristallisation mit 1%-iger HCl-IPA-MEK Mischung plus 5% Zitronensäure und zusätzlichem Waschen mit leichtflüchtigem etherischen Lösungsmittel + 5% Zitronensäure:
Das Tolperison-Rohprodukt wird in einer 85:15 Mischung aus 2-Butanon (MEK) und Isopropanol (IPA) und unter Zugabe von 1%-iger HCl und 5 Gew.% Zitronensäure unter Rückfluss für 12 Stunden gelöst. Die Temperatur wird auf 80°C gesenkt, und die Lösung heiß filtriert. Die Lösung wird auf 5°C abkühlen gelassen und 7h bei 5°C gerührt. Der kristalline Niederschlag wird abfiltriert, mit MEK, 0,5% HCl gewaschen, ein weiteres Mal mit der gleichen Volumenmenge an tert-Butylmethylether, 5% Zitronensäure gewaschen und anschließend im Vakuum bei 50°C getrocknet.

Durch Analyse mittels HPLC-MS/MS wird ein Anteil von 4-MMPPO in einem Bereich <1 ppm ermittelt.

Die Anteile an Piperidinhydrochlorid wurden mittels HPLC überprüft, wobei Werte von unterhalb 100 ppm bis 0 ppm (d.h. nicht nachweisbar) ermittelt wurden.

Erfindungsgemäße Variante Vb) Industrielles Verfahrensbeispiel: Umkristallisation mit 1%-iger HCl-IPA-MEK Mischung plus 5 % Zitronensäure und zusätzlichem Waschen mit leichtflüchtigem, etherischem Lösungsmittel + 5 % Zitronensäure:
165,4 kg Tolperison-Rohprodukt werden in einer 85:15 Mischung aus 2-Butanon (MEK, 900 kg) und Isopropanol (IPA, 75 kg) unter Zugabe von 1%-iger HCl (6,6 1) und 5 Gew.% Zitronensäure (8 kg) unter Rückfluss für 12 Stunden gelöst. Die Temperatur wird auf 80°C gesenkt und die Lösung heiß filtriert. Die Lösung wird auf 5°C abkühlen gelassen und 7 Stunden bei 5°C gerührt. Der kristalline Niederschlag wird abfiltriert, mit MEK (50 1), 0,5 % HCl gewaschen, ein weiteres Mal mit der gleichen Volumenmenge an tert-Butylmethylether, 5 % Zitronensäure gewaschen und anschließend im Vakuum bei 50°C getrocknet. Ausbeute 99,9 kg (60,4 %).

Durch Analyse mittels HPLC-MS/MS werden ein Anteil an 4-MMPPO in einem Bereich <1 ppm und ein Anteil von Piperidinhydrochlorid von <100 ppm ermittelt.

Der gemäß Ausführungsbeispiele gewonnene Wirkstoff Tolperison kann nach dessen Analyse in übliche Verfahren zur Herstellung von pharmazeutischen Formulierungen eingesetzt werden, wobei der Wirkstoff mit dem jeweiligen pharmazeutischen Träger vermengt, granuliert und tablettiert bzw. in diesen zur Ausbildung einer Suspension oder Sirups aufgenommen wird.

Zur Herstellung einer Filmtablette mit Instant-Release-Eigenschaften wird beispielsweise folgende Arbeitsvorschrift gewählt:
Beispiel 1: Tablettierung über Feuchtgranulation

Es wird eine Lösung aus wasserfreier Zitronensäure, Butanon und Isopropylalkohol hergestellt. Das erfindungsgemäß hergestellte Tolperison-Hydrochlorid wird in einen Granulator übergeführt, in welchen die bereits hergestellte Lösung eingebracht wird. Diese Mischung wird homogenisiert und anschließend in einem Trockner bei 60°C getrocknet. Das gebildete Granulat wird durch ein 1,8 mm Sieb gesiebt. Siliziumdioxid und Talcum werden hinzugegeben und ebenso vermischt. Anschließend wird mit Magnesiumstearat vermischt. Es werden Tabletten mit einem Durchmesser von 8 mm und einem Gewicht von 155,8-172,2 g hergestellt. Das fertige Granulat wird mit einer Suspension aus Hypromellose/Hypromellosephthalat in Ethanol/Wasser Farbstoffen und Zusätzen in einem Beschichtungskessel bei einer Temperatur von 55-60°C beschichtet. Die beschichteten Tabletten werden anschließend bei Raumtemperatur getrocknet.

### Beispiel 2: Direktverpressung

Es wird eine Mischung mit folgender Zusammensetzung hergestellt:

| Substanz | Anteil % |
|---|---|
| Tolperison | 78 |
| Zitronensäure, wasserfrei | 3 |
| Primojel | 2,5 |
| Super Tab 22 AN | 16 |
| Aerosil 200 | 0,5 |

Die Mischung wird mit einem 1,8 mm Sieb gesiebt und in einer Tablettenpresse direkt verpresst.

### Bespiel 3: Trockengranulierung

Es wird eine Mischung folgender Zusammensetzung hergestellt:

| Reagenz | Anteil % |
|---|---|
| Tolperison | 72 |
| Zitronensäure, wasserfrei | 5 |
| Stearinsäure | 0,5 |
| Super Tab 22 AN | 19,5 |
| Starch 1500 LM | 3 |

Der wirkstoff und eine Mischungskomponente werden in einem Trockenkompaktierer vorkompaktiert und anschließend zerkleinert. Das Granulat wird daraufhin mit einer Tablettenpresse zu Tablettenkernen verpresst.

Zur Ausbildung einer pharmazeutischen Formulierung mit kontrollierter Freisetzung kann beispielsweise nach folgender Rezeptur gearbeitet werden:

Racemisches Tolperison mit einem Anteil von weniger als 50 ppm 4-MMPPO wird zusammen mit einer Lösung aus Eudragit RS in 2-Butanon granuliert. Eudragit S und Eudragit L werden anschließend homogen eingerührt. Zu der Mischung gibt man 5 Gew.% Zitronensäure bezogen auf 100 Gew.% Tolperison und 4-MMPPO. Die Mischung wird getrocknet und gesiebt. Das gesiebte Granulierungsmaterial wird mit weiteren Zusatzstoffen, wie Retard-Vermittler gemischt und tablettiert, sodass der Tablettenkern erhalten wird. Dieser hat einen Durchmesser von etwa 8mm und ein Gewicht von 190 mg. Der Tablettenkern wird anschließend mit einem Film aus Eudragit L, Farbpigmenten und weiteren Zusatzstoffen, die in Butanol gelöst werden, beschichtet.

| Inhaltsstoff | Menge (mg) |
|---|---|
| Tolperison hydrochlorid | 150 |
| Eudragit RS | 1.88 |
| Eudragit L (Kern) | 10.50 |
| Eudragit L (Beschichtung) | 3.74 |
| Eudragit S | 10.50 |
| Aerosil | 1.80 |
| Stearinsäure | 1.80 |
| Glycerol Dibehenat | 7.50 |
| Eisenoxid (Pigment) | 0.08 |
| Titandioxid | 4.08 |
| Talcum | 6.03 |
| Polyethylenglycol | 1.02 |
| Dimethylpolysiloxan | 0.05 |
| Zitronensäure | 7,5 |

Die Verwendung von Säure beim Tablettierungsvorgang sowie das Arbeiten unter wasserfreien Bedingungen gewährleistet, dass der Anteil von 4-MMPPO zumindest beibehalten oder sogar zusätzlich reduziert wird.

Ein transdermales, therapeutisches System wird beispielsweise als Wirkstoffpflaster hergestellt, wobei der Wirkstoff Tolperison mit einem Anteil von weniger als 10 ppm 4-MMPPO und den Bestandteilen für die haftklebende Reservoirschicht, gegebenenfalls in Lösung homogen vermischt, auf die Wirkstoff undurchlässige Rückseite aufgestrichen wird, worauf gegebenenfalls das oder die Lösungsmittel entfernt werden. Anschließend wird die Klebeschicht mit einer entsprechenden Schutzschicht versehen.

Ein mögliches Herstellungsverfahren lässt sich wie folgt zusammenfassen:
Je 10,0 g Octansäure und 10,0g Isopropylmyristat werden unter Rühren gemischt. Anschließend werden 10,0 g Tolperison Hydrochlorid mit einem Anteil von weniger als 50 ppm eingetragen, wobei man bis zum vollständigen Auflösen des Feststoffes rührt. Danach werden 130,0g eines selbstvernetzenden Acrylatcopolymeren aus 2-Ethylhexylacrylat, Vinylacetat und Acrylsäure 46%-ig in einem Lösungsmittelgemisch (Ethylacetat:Heptan:Isopropanol:Toluol: Acetylaceton = 37:26:26:4:1) zugegeben; es wird homogenisiert. Danach werden unter Rühren noch zusätzlich 10g eines Methacrylatcopolymers auf Basis von Dimethylamonimethacrylat und neutralen Methacrylsäurestern eingestreut und 3 Stunden bei Raumtemperatur gerührt.

Es resultieren 150 g 52,8%-ige wirkstoffhaltige Kleberlösung, die auf eine aluminisierte und siliconisierte Polyethylenfolie gestrichen wird. Nach dem Trocknen bei 60 °C deckt man den Kleberfilm mit Polyesterfolie ab. Die einzelnen Wirkstoffpflaster werden mit einem geeigneten Schneidewerkzeug ausgestanzt.

Eine topische Formulierung enthaltend Tolperison mit einem Anteil von weniger als 10 ppm 4-MMPPO kann als Gel, Creme oder Salbe auf die Haut aufgetragen werden. Zur Erhöhung der Bioverfügbarkeit und gleichzeitiger Stabilisierung der Formulierung können Carbonsäuren als Zusatz verwendet werden. Dabei ist es möglich, den Wirkstoff in Kombination mit weiteren Schmerzmitteln einzusetzen.

Für die Herstellung eines Gels werden Wasser (145g) und eine Natriumhydroxidlösung (6g/5%-ige Lösung) vermischt. In diese Mischung wird ein Carbomer (1g) langsam eingerührt. Anschließend wird 2-Propanol (50g) eingerührt und der Wirkstoff in Form von Tolperison Hydrochlorid (2g) mit einem Anteil von weniger als 10 ppm 4-MMPPO eingerührt.

Für die Herstellung einer Creme werden Vaseline (50 g) und Cetylstearylalkohol (20 g) zusammen aufgeschmolzen bis eine klare Phase entsteht. Zeitgleich wird eine Mischung aus Polysorbat (10g), Glycerin (20g) und Wasser (98 g) plus Zitronensäure (3g) und Tolperison Hydrochlorid mit einem Anteil von weniger als 50 ppm 4-MMPPO (2g) verrührt und zu der aufgeschmolzenen Masse hinzugegeben und kaltgerührt.

Zur Herstellung der Tolperisonsalbe werden weißes Vaseline (92,5g), Cetylstearylalkohol (0,5g), Wollwachsalkohole (6g) Zitronensäure (1,5g) und Tolperisonhydrochlorid (1g) mit einem Anteil von weniger als 50 ppm 4-MMPPO zusammen aufgeschmolzen und kaltgerührt.

Zusammenfassend lässt sich sagen, dass durch das erfindungsgemäße Verfahren erstmalig der Anteil an 4-MMPPO im Hinblick auf die derzeit geltenden Richtlinien betreffend den Anteil von Nebenwirkungen verursachenden Stoffen in Arzneimittelwirkstoffen den zulässigen Höchstwert unterschreitet. Dies ist insofern von Bedeutung, da neueste Richtlinien der FDA und EMEA bezüglich genotoxischer Substanzen (Guideline on the limits of genotoxic impurities CPMP/SWP/5199/02) einen gewünschten Gehalt derselben von < 1,5 ig/Tag in arzneilichen Wirkstoffen vorschreiben und ein Überschreiten die neue Arzneimittelzulassung verhindert. Da im Fall von 4-MMPPO genotoxische oder carcinogene Wirkungen in Vitro nachgewiesen wurden und in Vivo daher auch vermutet werden, besteht die Anforderung, dass lediglich Werte im Bereich unterhalb von 10 ppm in einer pharmazeutischen Formulierung akzeptiert werden. Auch diese äußerst strengen Kriterien können erstmalig durch das erfindungsgemäße Verfahren sowie durch die wasserfreien Bedingungen beim Herstellen der pharmazeutischen Formulierung in Gegenwart zumindest e.ines wasserfreien Additivs, vorzugsweise wasserfreie Zitronensäure, erfüllt werden. Wird auch der für die Lagerung bestimmten Tolperison enthaltenden Wirkstoffformulierung ein saurer Stabilisator zugesetzt, so kann die unerwünschte Nebenreaktion unter Bildung von 4-MMPPO verhindert werden.

## Patentansprüche

1. Verfahren zur Herstellung von hoch reinem 2,4'-Dimethyl-3-piperidino-propiophenon (Tolperison), seiner pharmazeutisch annehmbaren Salze, Hydrochloride und Hydrate, bei dem 4-Methylpropiophenon 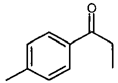 mit Piperidinhydrochlorid 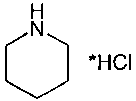 und 1,3-Dioxolan 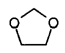 in Gegenwart einer oder mehrerer Säuren als Katalysator umgesetzt wird und das Tolperison-Rohprodukt in freier Form oder in Form seiner pharmazeutisch verträglichen Hydrochloride, Hydrate und Additionssalze nach dem Abkühlen aus der Reaktionsmischung abfiltriert wird und wobei durch Umkristallisation des Tolperison-Rohproduktes der Anteil an 2-Methyl-1-(4-methylphenyl)-propenon (4-MMPPO) 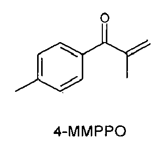 verkleinert wird, **dadurch gekennzeichnet, dass** beim Umkristallisieren des Tolperison-Rohproduktes eine Säure zugesetzt und der Anteil des 2-Methyl-1-(4-methylphenyl)-propenon (4-MMPPO) auf weniger als 10ppm bezogen auf 100-Gew.% Tolperison-Rohprodukt reduziert wird.

2. Verfahren nach Anspruch 1, **dadurch gekennzeichnet, dass** die Reaktion in Gegenwart katalytischer Mengen wässriger Chlorwasserstofflösung durchgeführt wird.

3. Verfahren nach Anspruch 1 oder 2, **dadurch gekennzeichnet, dass** die Reaktion in einem Lösungsmittel durchgeführt wird.

4. Verfahren nach Anspruch 3, **dadurch gekennzeichnet, dass** die Reaktion in 1,3-Dioxolan als Lösungsmittel in einem Konzentrationsbereich von 1 bis 6, vorzugsweise 3,6 mol/lit. durchgeführt wird.

5. Verfahren nach einem der Ansprüche 1 bis 4, **dadurch gekennzeichnet, dass** das Tolperison-Rohprodukt als Additionssalz durch Zugabe von wenigstens einem Gegenlösungsmittel, wie Ethylacetat und Methyl-tert.-butyl-ether, aus dem Reaktionsgemisch gefällt wird.

6. Verfahren nach einem der Ansprüche 1 bis 5, **dadurch gekennzeichnet, dass** das Umkristallisieren aus einer Lösungsmittelmischung bestehend aus einem Ether und einem Alkohol erfolgt.

7. Verfahren nach einem der Ansprüche 1 bis 6, **dadurch gekennzeichnet, dass** das Umkristallisieren in mehreren Stufen erfolgt.

8. Verfahren nach einem der Ansprüche 1 bis 7, **dadurch gekennzeichnet, dass** nach dem Umkristallisieren ein Trocknungsschritt, vorzugsweise eine Vakuumtrocknung erfolgt.

9. Verfahren nach einem der Ansprüche 1 bis 8, **dadurch gekennzeichnet, dass** beim Umkristallisieren ein Adsorptionsmittel, vorzugsweise Aktivkohle oder Silicagel, eingesetzt wird.

10. Verfahren nach einem der Ansprüche 1 bis 9, **dadurch gekennzeichnet, dass** Tolperison, dessen Salze, Hydrate und Hydrochloride als Racemat, als S- bzw.R-Enantiomer bzw. als chirale Mischung erhalten wird (werden).

11. Verfahren nach einem der Ansprüche 1 bis 10, **dadurch gekennzeichnet, dass** der Anteil an 2-Methyl-1-(4-methylphenyl)-propenon (4-MMPPO) auf weniger als 7 ppm reduziert wird.

12. Verfahren nach einem der Ansprüche 1 bis 10, **dadurch gekennzeichnet, dass** der Anteil an 2-Methyl-1-(4-methylphenyl)-propenon (4-MMPPO) auf weniger als 3 ppm reduziert wird.

13. Verfahren nach einem der Ansprüche 1 bis 10, **dadurch gekennzeichnet, dass** der Anteil an 2-Methyl-1-(4-methylphenyl)-propenon (4-MMPPO) auf weniger als 1,5 ppm oder auf 0 ppm (das heißt unterhalb der Nachweisgrenze) reduziert wird.

14. Verfahren nach einem der Ansprüche 1 bis 13, **dadurch gekennzeichnet, dass** der Anteil weiterer Verunreinigungen, insbesondere Piperidinhydrochlorid, auf kleiner 100 ppm, vorzugsweise 0 ppm (das heißt unterhalb der Nachweisgrenze) abgereichert wird.

## Claims

1. A process for synthesis of high-purity 2,4'-dimethyl-3-piperidino-propiophenone (tolperisone), its pharmaceutically acceptable salts, hydrochlorides and hydrates, in which 4-methylpropiophenone 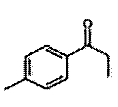 is reacted with piperidine hydrochloride 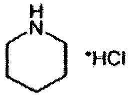 and 1,3-dioxolane 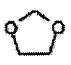 in the presence of one or more acids as catalysts, and after cooling, the tolperisone raw product is filtered out of the reaction mixture in free form or in the form of its pharmaceutically tolerable hydrochlorides, hydrates and addition salts, and the proportion of 2-methyl-1-(4-methylphenyl)-propenone (4-MMPPO) 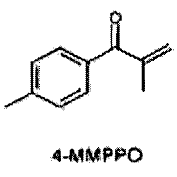 is reduced by recrystallizing the tolperisone raw product, **characterized in that** an acid is added in recrystallizing the tolperisone raw product and the amount of 2-methyl-1-(4-methylphenyl)-propenone (4-MMPPO) is reduced to less than 10 ppm based on 100 wt% tolperisone raw product.

2. The process according to claim 1, **characterized in that** the reaction is carried out in the presence of catalytic amounts of an aqueous solution of hydrogen chloride.

3. The process according to claim 1 or 2, **characterized in that** the reaction is carried out in a solvent.

4. The process according to claim 3, **characterized in that** the reaction is carried out in 1,3-dioxolane as the solvent in a concentration range from 1 mol/l to 6 mol/l, preferably 3.6 mol/l.

5. The process according to any one of claims 1 to 4, **characterized in that** the tolperisone raw product is precipitated from the reaction mixture as an addition salt by adding at least one counter solvent, such as ethyl acetate and methyl-tert-butyl ether.

6. The process according to any one of claims 1 to 5, **characterized in that** the recrystallization is carried out from a solvent mixture consisting of an ether and an alcohol.

7. The process according to any one of claims 1 to 6, **characterized in that** the recrystallization is carried out in several steps.

8. The process according to any one of claims 1 to 7, **characterized in that** after recrystallization, a drying step, preferably a vacuum drying is performed.

9. The process according to any one of claims 1 to 8, **characterized in that** an adsorbent, preferably charcoal or silica gel, is used in recrystallization.

10. The process according to any one of claims 1 to 9, **characterized in that** tolperisone, its salts, hydrates and hydrochlorides are obtained as racemate, as S-, respectively R-enantiomer, respectively as chiral mixture.

11. The process according to any one of claims 1 to 10, **characterized in that** the amount of 2-methyl-1-(4-methylphenyl) propenone (4-MMPPO) is reduced to less than 7 ppm.

12. The process according to any one of claims 1 to 10, **characterized in that** the amount of 2-methyl-1-(4-methylphenyl) propenone (4-MMPPO) is reduced to less than 3 ppm.

13. The process according to any one of claims 1 to 10, **characterized in that** the amount of 2-methyl-1-(4-methylphenyl) propenone (4-MMPPO) is reduced to less than 1.5 ppm or to 0 ppm (i.e., below the limit of detection).

14. The process according to any one of claims 1 to 13, **characterized in that** the amount of additional impurities, in particular piperidine hydrochloride, is reduced to less than 100 ppm, preferably to 0 ppm (i.e., below the limit of detection).

## Revendications

1. Procédé de préparation de 2,4'-diméthyl-3-pipéridino-propiophénone (tolpérisone) ayant un haut degré de pureté, de ses sels, hydrochlorures et hydrates pharmaceutiquement acceptables, consistant à faire réagir la 4-méthylpropiophénone 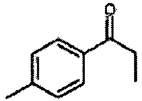 avec l'hydrochlorure de pipéridine 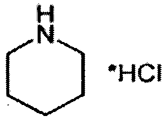 et le 1,3-dioxolane 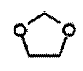 en présence d'un ou de plusieurs acides servant de catalyseur, et à récupérer la tolpérisone crue sous forme libre ou sous forme de ses hydrochlorures, hydrates et sels d'addition pharmaceutiquement compatibles en filtrant le mélange réactionnel après l'avoir laissé refroidir, et à diminuer la proportion de 2-méthyl-1-(4-méthylphényl)-propénone (4-MMPPO) 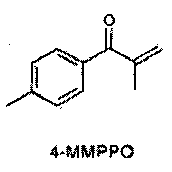 en soumettant la tolpérisone crue à une recristallisation, **caractérisé en ce que** la recristallisation de la tolpérisone crue comporte l'ajout d'un acide et que la proportion de 2-méthyl-1-(4-méthylphényl)-propénone (4-MMPPO) est diminuée de manière à représenter moins de 10 ppm par rapport à 100 % en poids de la tolpérisone crue.

2. Procédé selon la revendication 1, **caractérisé en ce que** ladite réaction est effectuée en présence de quantités catalytiques d'une solution aqueuse de chlorure d'hydrogène.

3. Procédé selon les revendications 1 ou 2, **caractérisé en ce que** ladite réaction est effectuée dans un solvant.

4. Procédé selon la revendication 3, **caractérisé en ce que** ladite réaction est effectuée dans du 1,3-dioxolane servant de solvant, sa concentration étant comprise entre 1 et 6 mol/l, la valeur préférée étant de 3,6 mol/l.

5. Procédé selon l'une des revendications 1 à 4, **caractérisé en ce que** la tolpérisone crue est précipitée à partir dudit mélange réactionnel sous forme d'un sel d'addition en y ajoutant au moins un contre-solvant tel que l'acétate d'éthyle et le méthyl tert.-butyl éther.

6. Procédé selon l'une des revendications 1 à 5, **caractérisé en ce que** ladite recristallisation est effectuée à partir d'un mélange de solvants constitué d'un éther et d'un alcool.

7. Procédé selon l'une des revendications 1 à 6, **caractérisé en ce que** ladite recristallisation est effectuée en plusieurs étapes.

8. Procédé selon l'une des revendications 1 à 7, **caractérisé en ce que** l'on effectue, suite à ladite recristallisation, une étape de séchage, s'agissant préférentiellement d'un séchage sous vide.

9. Procédé selon l'une des revendications 1 à 8, **caractérisé en ce que**, lors de ladite recristallisation, on met en oeuvre un adsorbant, s'agissant préférentiellement de charbon actif ou de gel de silice.

10. Procédé selon l'une des revendications 1 à 9, **caractérisé en ce que** la tolpérisone, ses sels, hydrates et hydrochlorures, est/sont obtenue/obtenus sous forme d'un mélange racémique, d'un énantiomère S ou R, ou bien d'un mélange de formes chirales.

11. Procédé selon l'une des revendications 1 à 10, **caractérisé en ce que** la proportion de 2-méthyl-1-(4-méthylphényl)-propénone (4-MMPPO) est diminuée de manière à représenter moins de 7 ppm.

12. Procédé selon l'une des revendications 1 à 10, **caractérisé en ce que** la proportion de 2-méthyl-1-(4-méthylphényl)-propénone (4-MMPPO) est diminuée de manière à représenter moins de 3 ppm.

13. Procédé selon l'une des revendications 1 à 10, **caractérisé en ce que** la proportion de 2-méthyl-1-(4-méthylphényl)-propénone (4-MMPPO) est diminuée de manière à représenter moins de 1,5 ppm ou à être égale à 0 ppm (c'est-à-dire inférieure au seuil de détection).

14. Procédé selon l'une des revendications 1 à 13, **caractérisé en ce que** la proportion d'autres impuretés, s'agissant notamment d'hydrochlorure de pipéridine, est réduite de manière à représenter moins de 100 ppm, préférentiellement à être égale à 0 ppm (c'est-à-dire inférieure au seuil de détection).
